# EUROPEAN PATENT SPECIFICATION

(11) **EP 3 804 803 B1**
(45) Date of publication and mention of the grant of the patent: **28.01.2026**
(21) Application number: 19202451.1
(22) Date of filing: 10.10.2019
(51) Int. Cl.: A61B 46/20, A61M 39/24, F16K 15/14, A61B 46/23

(54) **A VALVE FOR A POUCH FOR COLLECTING A FLUID FLOW AND A USE OF THE VALVE**
VENTIL FÜR EINEN BEUTEL ZUM SAMMELN EINES FLÜSSIGKEITSSTROMS UND VERWENDUNG DES VENTILS
SOUPAPE POUR UN SACHET POUR COLLECTER UN ÉCOULEMENT DE FLUIDE ET UTILISATION DE LA SOUPAPE

(43) Date of publication of application: 14.04.2021
(73) Proprietor: Serres Oy, 61850 Kauhajoki as. (FI)
(72) Inventor: Korkeamäki, Rami-Matti, 61850 KAUHAJOKI AS (FI); Mäkiranta, Jarmo, 61850 KAUHAJOKI AS (FI); Mäkiranta, Iiro, 61850 KAUHAJOKI AS (FI)
(74) Representative: Kolster Oy Ab

(56) References cited:
- WO-A1-2004/021912
- WO-A1-2005/007006
- US-A- 4 522 623
- US-A- 5 308 163
- US-A1- 2017 105 807

## Description

### FIELD OF THE INVENTION

The present invention relates to a valve for a pouch for collecting a fluid flow running along a surgical drape.

### BACKGROUND OF THE INVENTION

Document WO 2017070332 discloses a surgical drape with an attachable fluid control pouch. The lower portion of the pouch includes a drainage port, which is closed prior to use. The drainage port is opened to define fluid flow paths from inside the pouch to outside the pouch and drain fluids collected in the pouch during the medical procedure.

One of the problems associated with the above arrangement is that it is not possible to continuously remove fluid from the pouch since the drainage port requires constant opening and closing.

US 5308163 discloses a check valve for fluid containers and a method for manufacturing the same.

US 4522623 discloses a suction bottle for medicinal purposes.

US 2017/105807 discloses a surgical drape with attachable fluid control pouch and a geometric alignment feature.

WO 2004/021912 discloses a surgical drape having a fluid collection pouch with an inflatable rim.

WO 2005/007006 discloses a body split surgical drape for use in shoulder surgery. The body split surgical drape includes a pouch for collecting fluids.

### BRIEF DESCRIPTION OF THE INVENTION

An object of the present invention is to provide a valve for a pouch for collecting a fluid flow running along a surgical drape in order to overcome the above problem. The objects of the invention are achieved by a valve which is characterized by what is stated in the independent claims. The preferred embodiments of the invention are disclosed in the dependent claims.

The invention is based on the idea of continuously removing fluid from the pouch. An advantage of the valve of the invention is that vacuum remains constant although the pouch is empty.

The valve and the pouch are described in this text in a position in which they are during their use.

During surgical operations a patient is covered by a surgical drape so that only the site to be operated is uncovered. Fluids used at the operation site, such as irrigation fluids, or fluids from a patient, such as blood or tissue fluids, run along the surgical drape.

A pouch is used for collecting a fluid flow running along the surgical drape. The pouch has an open mouth such that the fluid flow is free to enter to the pouch. The upper rear part of the pouch may be attached to the surgical drape e.g. by an adhesive tape. The pouch has an open end so that the fluid flow can exit from the pouch. The pouch may comprise a filter or a screen for filtering solid particles, such as bone fragments.

The open end is provided with a valve. The valve comprises a closing mechanism. The valve comprises a body which may be formed of at least one plastic film. The body may be formed of a single tubular film, or two parallel films whose edges are joined together. The body has an input and an output. The input is preferably at the top of the body or near the top. The output is preferably at the bottom of the body or near the bottom. The body may have at least one static part for narrowing the path for the fluid flow between the input and the output. The output has preferably a flat structure inside the body. For example, the output may be a flanged bushing.

The valve may be inside the pouch or outside it. The valve is preferably disposable. When the valve is inside the pouch itself is utilized for forming the valve. The lower part of the pouch forms the valve, i.e. there is a body in the lower part of the pouch. The fluid flow enters to the body through the open end of the pouch which forms an input. Downstream from the input there may be at least one static part for narrowing the path for the fluid flow. The fluid flow follows the path which is not closed by the at least one static part. The static part for narrowing the path for the fluid flow is a part, which has no movable components, i.e. it limits the flow only by existing in a certain place. The at least static part may be a heat sealed joint which fuses the opposite walls of the pouch. There may be more than one heat sealed joint, e.g. two heat sealed joints. Instead of the heat-sealed joint there may be a two-sided adhesive tape inside the body, or a clip outside the body.

When the valve is outside the pouch the valve is a separate part which is connected to the pouch by a hose or a like. The valve comprises a body formed of at least one plastic film. The body has an input, at least one static part for narrowing the path for the fluid flow fluid downstream from the input and an output downstream from the at least one static part for narrowing the path for the fluid flow. The static part for narrowing the path for the fluid flow is a part, which has no movable components. The at least one static part may be a heat sealed joint which fuses the walls of the valve together. There may be more than one heat sealed joint, e.g. two heat sealed joints. The input and the output have preferably a flat structure inside the body. For example, the input and the output may be flanged bushings. The input and the output are preferably similar to each other so that the input can be used as the output and vice versa. When the input and the output are similar the advantage is that it is not possible to assemble the valve in a wrong way.

The body may have any form, it may be e.g. rectangular or circular.

It is possible that the body does not comprise the at least one static part for narrowing the path for the fluid flow. The above-mentioned option is relevant to both the valve outside the pouch and the valve inside the pouch. In that case the body shall be substantially narrow. For example, when the body has a rectangular shape its width may be at the highest 70 mm, preferably at the highest 60 mm and more preferably at the highest 50 mm. The width direction is transverse to the flow direction of the fluid.

The valve, either inside or outside of the pouch, works as follows: When fluid starts to flow through the pouch the valve opens and fluid runs through at least one flow path to the output of the valve. The output of the valve may be connected to a tube leading to a collection container, such as a collection canister or a collection liner, i.e. a suction bag. When the fluid flow runs dry the closing mechanism starts to work, e.g. the opposite films cling to each other due to the vacuum influencing through the output of the valve. When the opposite films cling to each other the valve closes. As a consequence vacuum remains substantially constant, i.e. the end of the fluid flow does not disturb suction in other tubes connected to the same suction channel. The at least one static part, such as the heat sealed joint, makes sure that vacuum suction is able to draw the opposite films together. Alternatively, the body of the valve is so narrow that vacuum suction is able to draw the opposite films together. When the fluid flow continues to the pouch the valve opens and fluid runs through the valve.

### BRIEF DESCRIPTION OF THE DRAWINGS

In the following the invention will be described in greater detail by means of preferred embodiments with reference to the accompanying drawings, in which
Figures 1a and 2a show front views of a pouch;
Figures 1b and 2b show cross sectional views of a pouch;
Figures 3a to 7a show front views of a valve;
Figures 3b to 7b show cross sectional views of a valve;
Figures 8 and 9 show front views of a pouch;
Figure 10a show a front view of a valve;
Figures 10b and 11 show cross sectional views of valves;
Figure 12 shows a schematic view of an apparatus for collecting fluid from above;
Figure 13 shows a schematic view of an arrangement for collecting fluid as a perspective view.

### DETAILED DESCRIPTION OF THE INVENTION

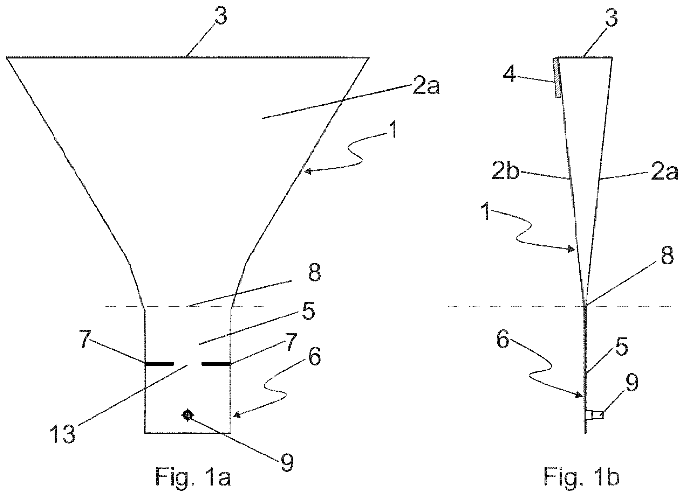

Figure 1 shows a pouch 1 and a valve 6 integrated in the pouch 1. The pouch 1 and the valve 6 comprise a front film 2a and a rear film 2b whose edges are seamed together. Another option is that there is a tubular film that has only a lower seam. The film / films forms/form a body 5. The pouch 1 is open from above, i.e. there is an open mouth 3. The rear film 2b may be provided with an adhesive tape 4. The pouch 1 has an open end which forms an input 8 for the valve 6. A dashed line in Fig. 1 separates the pouch 1 and the valve 6.

The valve 6 comprises at least one static part for narrowing the path for the fluid flow while the fluid flow enters in the valve 6. The static part is in this case heat sealed joints 7. Between the heat sealed joints 7 there is a flow path 13. Downstream from the heat sealed joints 7 there are the front film 2a and the rear film 2b whose edges are joined together. The valve 6 comprises an output 9 for removing fluid. The output 9 may be a flanged bushing.

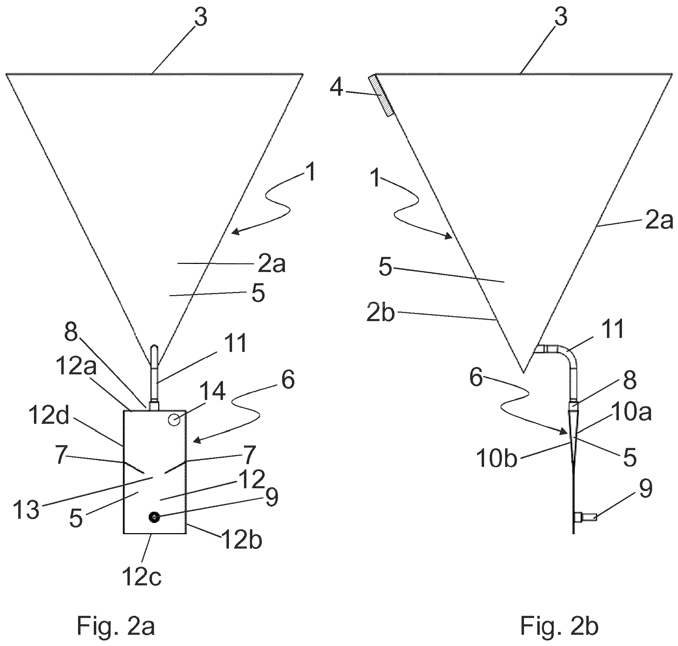

Figure 2 shows a pouch 1 and a separate valve 6. The pouch comprises a front film 2a and a rear film 2b whose edges are seamed together. The pouch 1 is open from above, i.e. there is an open mouth 3. The rear film 2a may be provided with an adhesive tape 4. The pouch 1 has an open end to which a hose 11 can be connected.

The valve 6 comprises at least one static part for narrowing the path for the fluid flow while the fluid flow enters in the valve 6. The static part is in this case heat sealed joints 7. Between the heat sealed joints 7 there is a flow path 13. The the valve 6 comprises a front film 10a and a rear film 10b. The edges 12a, 12b, 12c, 12d of the front film 10a are joined to the respective edges of the rear film 10b. Another option is that there is a tubular film that has only an upper and lower seam, i.e. only the edges 12a, 12c are joined to the respective edges of the rear film 10b. The film / films forms/form a body 5. The valve 6 comprises an output 9 for removing fluid.

The valve 6 may comprise an opening 14 such that the valve is able to draw air from outside of the valve 6. The opening 14 may be used if the fluid flows gravitationally. Instead of the opening there may be a dashed line showing where to cut in order to form the opening 14 if required. The opening makes possible to remove air from the fluid flow and thus, the collection container, such as a suction bag, fills up with fluid.

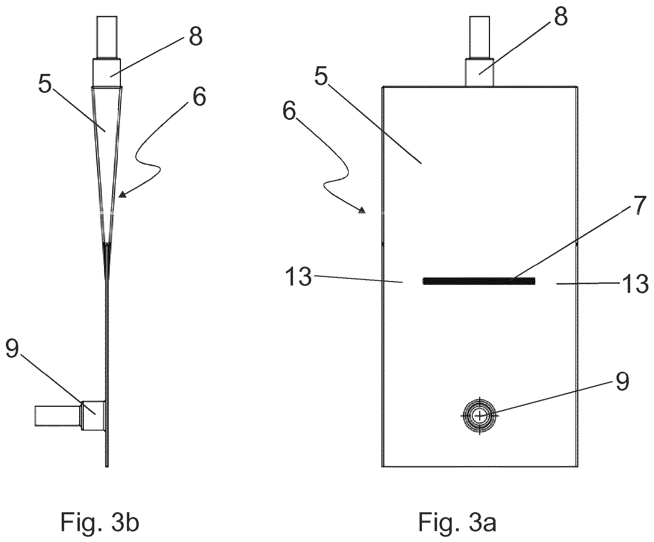

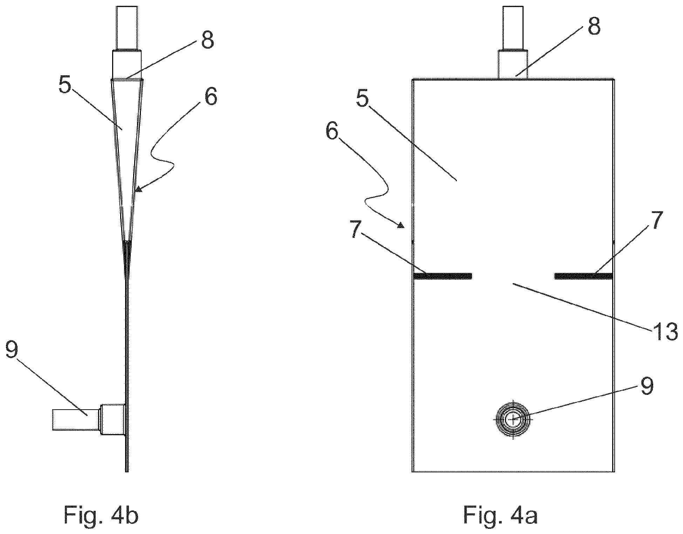

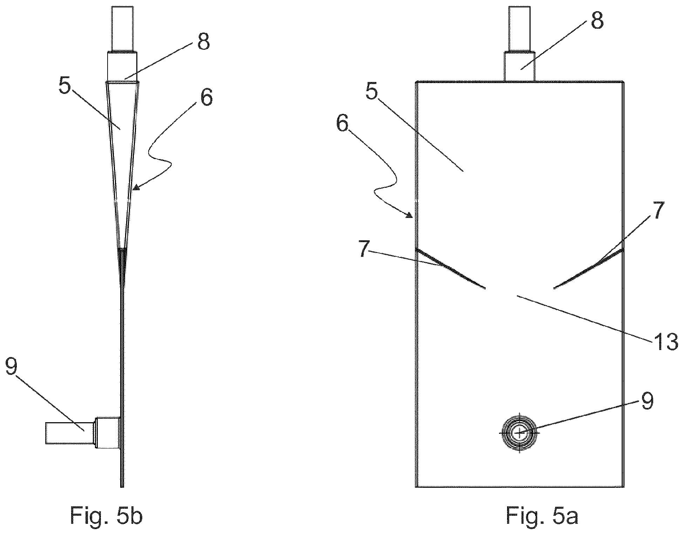

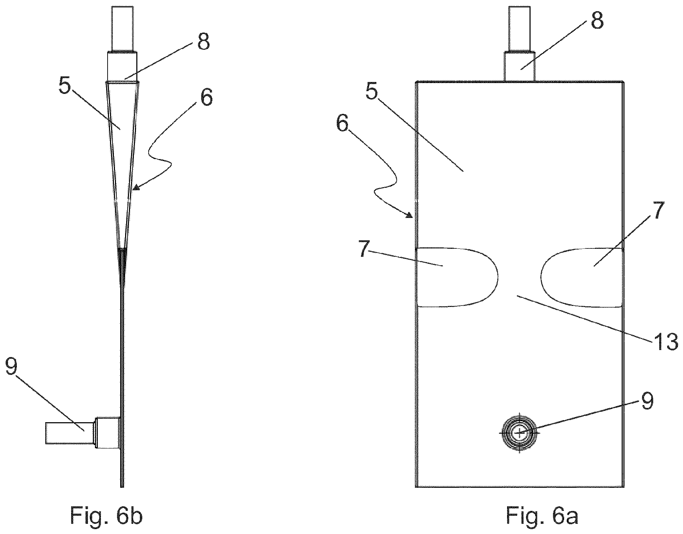

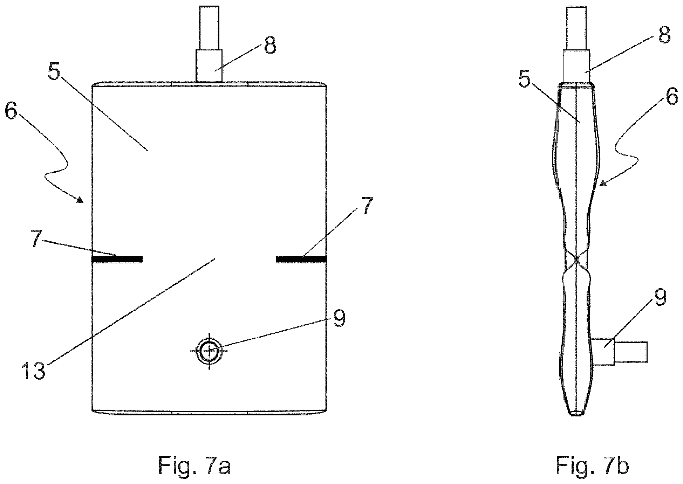

Figures 3 to 7 show variations of the separate valve 6. The valve of Fig. 3 has only one heat sealed joint 7 as the static part for narrowing the path for the fluid flow. Thus, there are two flow paths 13. The valve of Fig. 4 has two straight heat sealed joints 7 as the static part for narrowing the path for the fluid flow. The valve of Fig. 5 has two diagonal straight heat sealed joints 7 as the static part for narrowing the path for the fluid flow. The valve of Fig. 6 has heat sealed joints 7 that are larger than the linear heat sealed joints. An advantage of these kind of joints is that they prevent the valve 6 from twisting around the vertical axis of the valve 6. The valve of Fig. 7 has two straight heat sealed joints 7 as the static part for narrowing the path for the fluid flow. The difference between the valve of Fig. 4 and the valve of Fig. 7 is that the valve of Fig. 4 is flat whereas the valve of Fig. 7 is rounder.

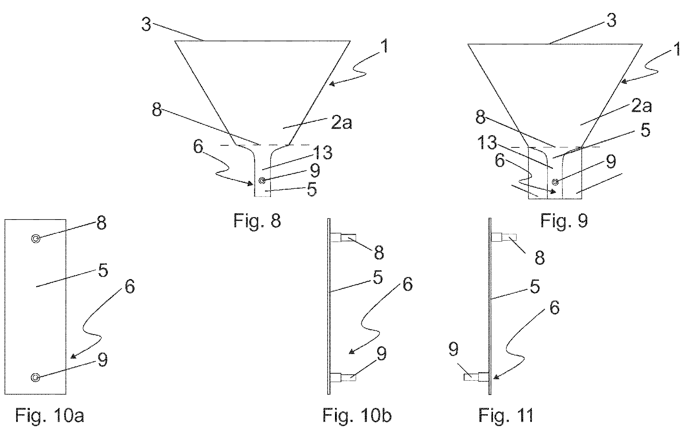

Figure 8 shows a pouch 1 and a valve 6 integrated in the pouch 1. The pouch 1 and the valve 6 comprise a front film 2a and a rear film 2b (not shown) whose edges are seamed together. Another option is that there is a tubular film that has only a lower seam. The film / films forms/form a body 5. The pouch 1 is open from above, i.e. there is an open mouth 3. The rear film 2b may be provided with an adhesive tape 4 (not shown). The pouch 1 has an open end which forms an input 8 for the valve 6. A dashed line in Fig. 8 separates the pouch 1 and the valve 6.

The valve 6 is narrow. For example, when the body 5 has a rectangular shape its width may be at the highest 70 mm, preferably at the highest 60 mm and more preferably at the highest 50 mm. Along the valve 6 there is a flow path 13. The valve 6 comprises an output 9 for removing fluid. The output 9 may be a flanged bushing.

Figure 9 shows a pouch 1 and a valve 6 integrated in the pouch 1. The pouch 1 and the valve 6 comprise a front film 2a and a rear film 2b (not shown) whose edges are seamed together. Another option is that there is a tubular film that has only a lower seam. The film / films forms/form a body 5. The pouch 1 is open from above, i.e. there is an open mouth 3. The rear film 2b may be provided with an adhesive tape 4 (not shown). The pouch 1 has an open end which forms an input 8 for the valve 6. A dashed line in Fig. 9 separates the pouch 1 and the valve 6.

The valve 6 is narrow. For example, when the body 5 has a rectangular shape its width may be at the highest 70 mm, preferably at the highest 60 mm and more preferably at the highest 50 mm. The narrowing is formed by heat sealing the opposite films of the valve 6 together. Along the valve 6 there is a flow path 13. The valve 6 comprises an output 9 for removing fluid. The output 9 may be a flanged bushing.

Figures 10a, 10b and 11 show a separate valve 6. The valve 6 has an input 8. The valve 6 does not comprise at least one static part for narrowing the path for the fluid flow 6 but the body 5 is throughout narrow. For example, the width of the body may be at the highest 70 mm, preferably at the highest 60 mm and more preferably at the highest 50 mm. The body 5 may have various lengths. Along the body 5 there is a flow path 13. The valve 6 comprises an output 9 for removing fluid. The input 8 and the output 9 may be flanged bushings. It is possible to use the valve 6 in both directions, i.e. either of the inlets can act as the input 8 or the output 9.

As the body 5 is narrow the vacuum suction is able to draw the opposite films of the body together and close the valve 6.

Fig. 10b show one option where the input 8 and the output 9 are on the same side of the body 5 whereas in Fig. 11 they are on the opposite side of the body 5.

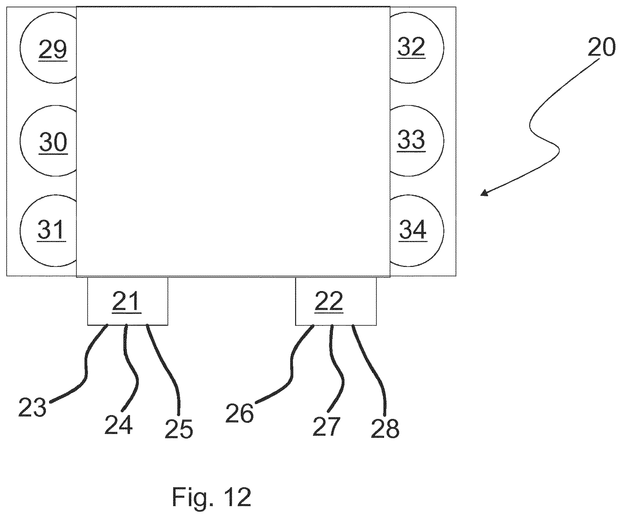

Figure 12 shows a schematic view of an apparatus 20 for collecting fluid during a medical or surgical operation. The valve 6 may be used with the apparatus 20. The apparatus 20 may be a one-part apparatus, or it may comprise a control unit and a movable cart.

The apparatus 20 comprises at least two individually operating suction channels. Each of the suction channels are provided with a disposable manifold. In a case of a two-part apparatus the manifold may be in the movable cart. A manifold 21 is connected to the first suction channel and a manifold 22 is connected to the second suction channel. Each of the suction channels are connected to a vacuum pump of the apparatus 20, or a vacuum system of a hospital or a like.

Tubes 23, 24, 25 are connected to the manifold 21 and tubes 26, 27, 28 are connected to the manifold 22. Inlets of the manifold 21 are provided with valves so that it is possible to direct the fluid flow to any of collection containers 29, 30, 31. Similarly, inlets of the manifold 22 are provided with valves so that it is possible to direct the fluid flow to any of collection containers 32, 33, 34. Each valve of the manifolds 21, 22 are operated by a suitable actuator, such as a motor. The collection containers 29, 30, 31, 32, 33, 34 may be collection canisters, collection liners, or canisters provided with collection liners, i.e. suction bags.

For example, the tube 23 may be for collecting fluid from a patient, the tube 24 may be for collecting fluid from a floor of an operating theatre and the tube 25 may be for collecting fluid from the pouch 1. As tubes 23, 24, 25 are connected to the same suction channel a lack of vacuum in any of the tubes 23, 24, 25 disturbs vacuum in the other tubes 23, 24, 25. If the pouch 1 is used without the valve 6 such a lack of vacuum takes place when the pouch 1 is empty and the tube in question is in direct contact with the atmospheric pressure.

The lack of vacuum can be prevented by the valve 6 of the invention. When the fluid flow runs dry the opposite films of the valve 6 cling to each other and close the valve 6. Thus, the original level of the negative pressure is maintained and the other tubes of the same suction channel operate without troubles.

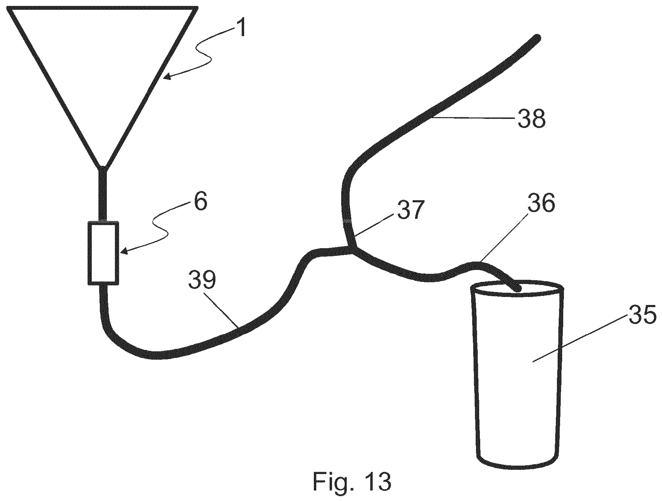

Figure 13 shows a schematic view of an arrangement that is possible in collecting fluid. A collection container 35, such as a suction bag, i.e. a collection liner or a collection canister, is provided with vacuum. A tube 36 is connected to the collection container 35. The tube branches by a Y connector 37 to a tube for collecting fluid 38 and a pouch tube 39. The tube for collecting fluid 38 may draw fluid from a patient, or it may be used for e.g. drying a floor of an operation theatre.

As the tube for collecting fluid 38 and the pouch tube 39 are connected to the same vacuum source the negative pressure in the tube 38 is affected by the atmospheric pressure when there is no fluid in the pouch tube 39. The lack of vacuum can be prevented by the valve 6 of the invention. When the fluid flow runs dry the opposite films of the valve 6 cling to each other and close the valve 6. Thus, the original level of the negative pressure is maintained and the tube for collecting fluid 38 connected to the same vacuum source operates without troubles.

The scope of the invention is solely defined by the appended claims.

## Claims

1. A valve (6) for a pouch (1) for collecting a fluid flow running along a surgical drape, **characterized in that** the valve (6) comprises a body (5), the body (5) is formed of opposite plastic films, the body is configured to have a path (13) for the fluid flow through the body (5), the body (5) has an input (8) and an output (9) for connecting a tube downstream from the input (8), the valve (6) comprises a closing mechanism that comprises the opposite plastic films.

2. The valve according to claim 1, **characterized in that** the valve (6) comprises at least one static part for narrowing the path (13) for the fluid flow between the input (8) and the output (9).

3. The valve according to claim 2, **characterized in that** the at least one static part for narrowing the path for the fluid flow is a heat sealed joint (7).

4. The valve according to claim 2 or 3, **characterized in that** the valve comprises two heat sealed joints (7).

5. The valve according to any preceding claim, **characterized in that** the valve (6) is integrated in the pouch (1).

6. A use of a valve (6) according to any preceding claim with a pouch (1) for collecting a fluid flow running along a surgical drape, the pouch (1) having an open mouth (3) for the fluid flow entering to the pouch (1) and an open end for the fluid flow exiting from the pouch (1) to the valve (6).

## Patentansprüche

1. Ventil (6) für einen Beutel (1) zum Sammeln einer Fluidströmung, die entlang eines Operationsabdecktuchs verläuft, **dadurch gekennzeichnet, dass** das Ventil (6) einen Körper (5) umfasst, wobei der Körper (5) aus gegenüberliegenden Kunststofffilmen besteht, wobei der Körper dazu ausgestaltet ist, einen Pfad (13) für die Fluidströmung durch den Körper (5) aufzuweisen, wobei der Körper (5) einen Eingang (8) und einen Ausgang (9) zum Verbinden eines Rohrs stromabwärts des Eingangs (8) aufweist, wobei das Ventil (6) einen Schließmechanismus umfasst, der die gegenüberliegenden Kunststofffilme umfasst.

2. Ventil nach Anspruch 1, **dadurch gekennzeichnet, dass** das Ventil (6) mindestens einen statischen Teil zum Verschmälern des Pfads (13) für die Fluidströmung zwischen dem Eingang (8) und dem Ausgang (9) umfasst.

3. Ventil nach Anspruch 2, **dadurch gekennzeichnet, dass** der mindestens eine statische Teil zum Verschmälern des Pfads für die Fluidströmung eine heißversiegelte Verbindung (7) ist.

4. Ventil nach Anspruch 2 oder 3, **dadurch gekennzeichnet, dass** das Ventil zwei heißversiegelte Verbindungen (7) umfasst.

5. Ventil nach einem der vorhergehenden Ansprüche, **dadurch gekennzeichnet, dass** das Ventil (6) in den Beutel (1) integriert ist.

6. Verwendung eines Ventils (6) nach einem der vorhergehenden Ansprüche mit einem Beutel (1) zum Sammeln einer Fluidströmung, die entlang eines Operationsabdecktuchs verläuft, wobei der Beutel (1) eine offene Mündung (3) für die Fluidströmung, die in den Beutel (1) eintritt, und ein offenes Ende für die Fluidströmung aufweist, die aus dem Beutel (1) zum Ventil (6) austritt.

## Revendications

1. Valve (6) pour un sachet (1) afin de collecter un écoulement de fluide s'étendant le long d'un champ opératoire, **caractérisée en ce que** la valve (6) comprend un corps (5), le corps (5) est formé avec des films en plastique opposés, le corps est configuré pour avoir une trajectoire (13) pour l'écoulement de fluide à travers le corps (5), le corps (5) a une entrée (8) et une sortie (9) pour raccorder un tube en aval de l'entrée (8), la valve (6) comprend un mécanisme de fermeture qui comprend les films en plastique opposés.

2. Valve selon la revendication 1, **caractérisée en ce que** la valve (6) comprend au moins une partie statique pour rétrécir la trajectoire (13) pour l'écoulement de fluide entre l'entrée (8) et la sortie (9).

3. Valve selon la revendication 2, **caractérisée en ce que** la au moins une partie statique pour rétrécir la trajectoire pour l'écoulement de fluide est un joint thermosoudé (7).

4. Valve selon la revendication 2 ou 3, **caractérisée en ce que** la valve comprend deux joints thermosoudés (7).

5. Valve selon l'une quelconque des revendications précédentes, **caractérisée en ce que** la valve (6) est intégrée dans le sachet (1).

6. Utilisation d'une valve (6) selon l'une quelconque des revendications précédentes avec un sachet (1) pour collecter un écoulement de fluide s'étendant le long d'un champ opératoire, le sachet (1) ayant une bouche ouverte (3) pour l'écoulement de fluide entrant dans le sachet (1) et une extrémité ouverte pour l'écoulement de fluide sortant du sachet (1) vers la valve (6).
